(19) Europäisches Patentamt
European Patent Office
Office européen des brevets

(11) Publication number: **0 089 939**
**A1**

# (12) EUROPEAN PATENT APPLICATION

(21) Application number: 83850077.5

(22) Date of filing: 22.03.83

(51) Int. Cl.[3]: A 61 K 31/70
C 07 H 3/04, A 61 K 39/00
G 01 N 33/48

(30) Priority: 22.03.82 SE 8201815

(43) Date of publication of application: 28.09.83 Bulletin 83/39

(84) Designated Contracting States: AT BE CH DE FR GB IT LI NL SE

(71) Applicant: Svenska Sockerfabriks AB
Box 17050
S-200 10 Malmö(SE)

(72) Inventor: Mardh, Per Anders
Holmgangen 13
S-223 75 Lund(SE)

(72) Inventor: Svensson, Sigfrid
Södra Ljungvägen 10
S-244 02 Furulund(SE)

(74) Representative: Burman, Tore et al,
Bergling & Sundbergh AB P.O. Box 7645
S-103 94 Stockholm(SE)

## (54) Compositions for therapeutic or diagnostic use containing oligosaccharides.

(57) A composition for therapeutic or diagnostic use in connection with disease-generating microorganisms, the composition containing as an active constituent a structural element having the formula:

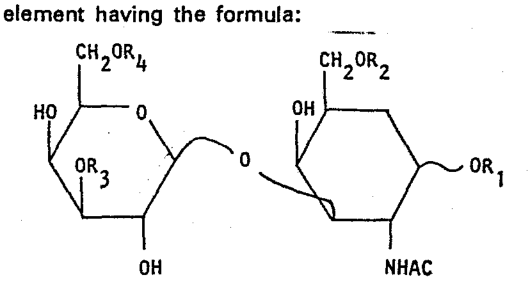

V

wherein $R_1$, $R_2$, $R_3$ and $R_4$ are same or different and are hydrogen or an organic residue, for example lower alkyl, lower acyl or a carbohydrate residue, or an inorganic residue, such as sulphate or phosphate, with the proviso that when $R_1$ is other than hydrogen $OR_1$ is in alpha-configuration; a process for therapeutic treatment; a process for identification or quantification of the structural element; a process for the purification of the acceptor structure of bacteria; and a process for disinfection.

EP 0 089 939 A1

0089939

TITLE MODIFIED
see front page

TITLE OF INVENTION

COMPOSITIONS FOR THERAPEUTIC OR DIAGNOSTIC USE AND A PROCESS FOR THERAPEUTIC TREATMENT

The present invention relates to compositions which can be used for therapeutic treatment of infections caused by disease-generating microorganisms, particularly pathogenic bacteria, such as Gram-negative cocci, particularly Gonococci and Meningococci, preferably of the Genus Meisseria, such as N. gonorrhoeae and N. meningitidis. The compositions can also be used for prophylactic and diagnostic processes. Moreover, the invention relates to a process for therapeutic treatment of mammals including man.

Many bacterial infection courses are initiated by the fact that the bacterium has ability to adhere to epithelium. This adhering capacity is specific in that different bacteria adhere preferentially to different types of epithelial tissues. Thus, studies have shown that the bacterium N. gonorrhoeae shows adhering specificity to vaginal cells (Ref. 1). It has also been shown that the adhering ability of the bacterium (N.gonorrhoeae) is dependent on pili (fimbriae) (Ref. 2). Pili are constituted by protein structures having the ability of recognizing some receptor structure on the surface of the epithelial cell. In addition to the fact that N-gonorrhoeae adheres to vaginal cells it can also agglutinate human erythrocytes independent of blood group.

The present invention has for its purpose to provide a composition or substance having the ability of replacing the normal receptor in vivo in relation to pathogenic bacteria prone to provide infections in man and animals.

Another object of the invention is to provide such composition or substance which, in addition to the therapeutic use, also can be used diagnostically.

A further object of the invention is to provide a process for identification and/or quantification of the receptor structure of native biologic materials from mammals including man. The invention also relates to a process for purification of bacteria or the acceptor structure of bacteria.

The invention is particularly directed to the receptor structure of N. gonorrhoeae and N. meningitidis, but it should be noted that the invention is not limited to these particular bacteria.

In connection with studies and experiments leading to the present invention it has been found that the receptor for N.gonorrhoeae and N. meningitidis found on the surface of epithelial cells and erythrocytes contains a structural element having the formula:

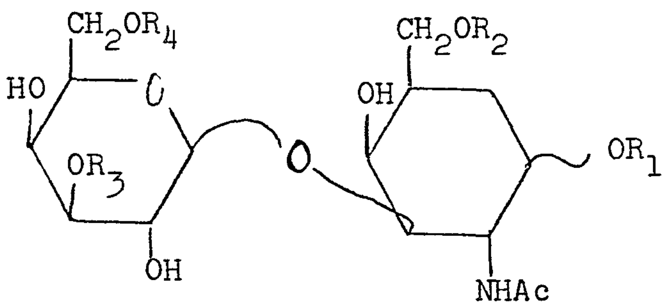

V

wherein $R_1$, $R_2$, $R_3$ and $R_4$ are same or different and are hydrogen or an organic residue, for example lower alkyl, lower acyl or a carbohydrate residue, or an inorganic residue, such as sulphate or phosphate, with the proviso that when $R_1$ is different from hydrogen $OR_1$ is in alpha-configuration, and that when $R_2=R_3=R_4=H$ $R_1$ is different from $-(CH_2)_nCOR$, where n=3-19 and R is an alkoxy, aryloxy, $NHNH_2$, OH or $N_3$ group.

In this structural formula $R_2$, $R_3$ and $R_4$ are preferably all hydrogen, a particularly preferred structural element thus having the formula:

β - D - Galp - (1-3) - α - D - GalNAcp

$R_1$ is suitably an amino acid or a polypeptide residue. According to another embodiment $R_1$ may be lower alkyl, for example methyl.

The active component in the composition according to the invention thus contains as an active constituent a structural element suitably having the formula:

β - D - Galp - (1-3) - α - D - GalNAcp - (1-O) - R

In this formula symbol R relates to an organic or inorganic residue, which is attached in α-configuration and which is of an arbitrary type as long as it does not adversely effect the circumstances in connection with the use of the composition.

The said structural element having the above formula is preferably positioned in terminal position, i.e. with the left end of the formula in freely exposed condition.

Other preferred structural elements are clear from the following named compounds showing the desired receptor activity as disclosed in the present disclosure.

The invention also relates to a process for therapeutic treatment of mammals including man, an active amount of the composition according to the invention being administered to the mammal.

According to another aspect of the invention there is provided a composition which can be used for identification and/or quantification of the said receptor structures in biological material or to purify bacteria or their receptor structures. The composition contains one or several structural elements according to the above covalently or otherwise linked to for example a macromolecular carrier, optionally via a coupling arm. Useful carriers may be synthetically or naturally occurring polypeptides, polysaccharides or other types of polymers or particles. This is conventional in the

art, see for example references 4-8, the contents of which are incorporated herein by reference.

Preferred structural elements of this type of composition according to the invention are defined above.

The active constituents according to the present invention may, in accordance with traditional pharmaceutical practice, be formulated for use in human medicine for therapeutic, prophylactic or diagnostic purposes. Such pharmaceutical preparations include the active constituent in combination with a pharmaceutically acceptable carrier, which may be solid, semisolid or liquid, and in some cases the active constituent according to the invention can be used as such without dilution.

The compositions of the invention include those in a form adapted for oral or parenteral use may be used for the treatment of mammals including humans.

Suitable forms of the compositions of this invention include tablets, capsules, syrups, suspensions, solutions, reconstitutable powders and sterile forms suitable for injection or infusion. Such compositions may contain conventional pharmaceutical acceptable materials, such as diluents, binders, colours, flavours, preservatives, disintegrants and the like in accordance with conventional pharmaceutical practice in the manner well understood by those skilled in the art of formulating drugs.

Injectable or infusable compositions of a structural element of the invention are particularly suitable as useful blood levels of the structural element can occur after administration by injection or infusion.

Several of the active structures according to the invention are previously known from the literature but they have not earlier been disclosed as possessing medicinally useful properties. To the extent the compounds described in the following are new and novel

the process for their manufacture is described. Such compounds of the invention known per se form a particular aspect of the invention.

Haemagglutination reaction of sheep erythrocytes and human cells independent of ABO-status which fimbriated strains of N. gonorrhoeae cause is probably due to interaction between the fimbriae of the bacterium and receptors of erythrocytes from sheep or man including the above-mentioned structural element.

The adherence of N. gonorrhoeae to vaginal cells is probably due to interaction between the fimbriae of the bacterium and receptors on the surfaces of the epithelial cells containing the above-mentioned structural elements.

The invention will in the following be described further by non-limiting examples.

EXAMPLE 1

Inhibition of haemagglutination of erythrocytes from sheep and man with fimbriated strain of N. gonorrhoeae, by addition of oligosaccharides.

In the test there is used a strain of N. gonorrhoeae (international reference strain 3 from Neisseriaavd. Statens seruminstitutt, Copenhagen, Denmark. and a fresh strain of the same species isolated at the Bacteriological Central Laboratory, Lunds Hospital, Lund, Sweden). The bacteria were cultivated on a special gonococc medium, see Mårdh, P.A., Mårtensson, D, Soltesz, L.V. "An effective, simplified medium for the culture of N. gonorrhoeae". Sex.Trans.Dis. Vol. 5, pp. 10-13, 1978, at + 37$^{o}$C for 18 hours. The bacterial culture was slurried in PBS, pH 7.2 to a density of 109 bacteria/ml.

Haemagglutination was performed using erythrocytes from sheep or man ($O_1$Rh+) which were washed with PBS and suspended to a concentration of 1 % v/w in PBS, pH 7.2. The agglutination tests were performed so that 25 µl of

the bacterial suspension was titrated in PBS by using two-fold fibre steps. Then 25 µl of the erythrocyte suspension which had been made 1 % were added. The haemagglutination titre (Hu) was determined after incubating the plates (microtitre plates, Limbo Sc. Comp. Inc.) for 2-4 hours in room temperature.

In the haemagglutination-inhibition tests there were used serial dilutions having the relevant inhibitor (50 µl/well) and 25 µl bacterial suspension diluted to contain twice the lowest haemagglutination titre. The plates were incubated for 30 minutes at +37°C, 25 µl of erythrocyte suspension being then added to each well. The result was read after 2-4 hours incubation at room temperature.

The results are presented in tables I and II.

TABLE I

Inhibition of haemagglutination of human erythrocytes ($O_1$Rh+) with N.gonorrhoeae by adding inhibitors.

| Inhibitor | Minimum concentration of inhibitor resulting in complete inhibition µg/ml |
|---|---|
| Asialofetuin (calf) | 1.5 |
| Asialo M-substance (human) | 3 |
| Asialo N-substance (human) | 3 |
| Asialo antithrombin III (human) | >1250 |
| β-D-Galp-(1-3)-D-GalNAc | 800 |
| β-D-Galp-(1-3)-β-D-GalNAcp-(1-O)Me$^{x)}$(6) | >2000 |
| β-D-Galp-(1-3)-α-D-GalNAcp-(1-O)Me$^{x)}$(3) | 200 |
| β-D-Galp-(1-3)-D-Gal | >5000 |
| β-D-Galp-(1-4)-D-Glc | >5000 |
| β-D-Galp-(1-6)-D-Glc | >5000 |

Table I cont.

| | |
|---|---|
| β-D-GalNAc-(1-3)-α-D-Galp-(1-4) -β-D-Galp-(1-4)-D-Glc | >5000 |

x) Novel compounds see encl. 1 concerning preparation.

TABLE II

Inhibition of haemagglutination of sheep erythrocytes with N. gonorrhoeae by adding inhibitors.

| Inhibitor | Minimum concentration of inhibitor resulting in complete inhibition of haemagglutination µg/ml |
|---|---|
| Asialofetuin (calf) | 6 |
| β-D-Galp-(1-3)-D-GalNAc | 900 |
| β-D-Galp-(1-3)-α-D-GalNAc-(1-O)Me | 150 |
| β-D-Galp-(1-3)-β-D-GalNAc-(1-O)Me | > 2000 |

EXAMPLE 2

Adherence studies with N.gonorrhoeae and vaginal cells and inhibition with oligosaccharides

Materials:

Cells: Vaginal cells from healthy women in fertile age not having used hormonal preparations and werein proliferation phase were tested. Cells were collected from rear vaginal fornix with a wooden spatula and suspended in RPMI 1640 in a concentration of $10^5$ cells/ml.

Gonococci: International reference strain 3 from Neisseriaavd. Statens Seruminstitut, Copenhagen, Denmark, suspended in cell cultivation medaium RPMI 1640 (Flow Ltd.) in a concentration of $10^9$ bacteria/ml.

Oligo-
saccharides: β-D-Galp-(1-3)- -D-GalNAc-(1-O)-Me (1)
β-D-Galp-(1-3)- -D-GalNAc-(1-O)-Me (2)

<u>Procedure</u>: 1 ml of RPMI 2640 containing 0 of (1), 5 mg/ml of (1) and 0.5 mg/ml of (1) and 5 mg/ml of (2), respectively, were incubated with 0.5 ml bacterial suspension at 37°C for 30 minutes. Vaginal cells (1 ml) were incubated with the above preincubated bacterial suspensions at 37°C for 45 minutes. The incubated suspensions were filtered through a filter (pore size 12 μ) and washed 3 times with PBS. The filter was pressed against microscope slides for a few seconds, the microscope slides being then fixed in methanol for 10 minutes. The microscope slides were dried in air and coloured with acridine orange for 2 minutes. After washing and drying in air the slides were studied by means of fluorescence microscopy. The number of bacteria adhering per cell was counted. In each test 50 vaginal cells were counted. It was found that 0.5 mg/ml of (1) and 5 mg/ml of (2) did not significantly effect adherence, whereas 5 mg/ml of (1) reduced the number of adhering bacteria by 87 %.

EXAMPLE 3

Preparation of compositions containing the structural element in at least bivalent state and covalently linked to a macromolecular carrier.

a. Glycoproteins containing the structural element covalently linked to serine or treonine and glycoproteins containing the structural element covalently linked to serine or treonine but wherein the structural element is substituted with one or several N-acetyl or N-glycolyl-neuraminic acid esters, for example k-casein, submaxillary mucines, fetuin, M-substance, N-substance, freezing point depressing glycoproteins in mucus from arctic fishes and glycoproteins in urine where desialylated using mild acid hydrolysis or neuraminidase treatment. The resulting glycoproteins have the structural element

β-D-Galp-(1-3)-D-GalNAcp-α-glycosidically linked to themselves.

b. The desialylated glycoproteins from a. are treated extensively with pronas and/or papin, glycopepsides being obtained. Using known techniques, for example DCC-coupling, these can be attached to different other macromolecular carriers.

EXAMPLE 4

Preparation of compositions containing the structural element according to the invention in at least bivalent state in association with other materials.

Desialylated glycoproteins according to Example 3 were made using hydrophobic interaction to lipophilic gels, polymers or particles, for example octyl-sepharose, plastics and latex surfaces.

EXAMPLE 5

Preparation of antibodies showing specificity towards the said structural element.

a. Preparation of monoclonal antibodies by hybridoma technique.

I. Balb/c-mice were immunized with a composition according to the invention. The spleen from hyperimmunized animal is harvested and a cell suspension is prepared by mechanical comminution of the tissue. After gradient centrifugation to obtain a pure cell preparation the cells are fused by means of polyethylene glycol (PEG average molecular weight 1500) with established B-myeloma cell lines from Balb/c mice according to known technique. After cloning of the hybridoma cells generating the antibody desired, the cells are propagated on a large scale, the culture medium supernatants being harvested and the antibodies are being purified in a conventional manner. For identifying antibody generating clones there is used so-called enzyme-linked immunosorbent assay (ELISA) (Ref. 5).

II. Mammals are immunized with an oligosaccharide protein or polymer composition according to the invention. Antibodies are isolated from the huperimmun serum of the mammal and purified in accordance with conventional techniques.

EXAMPLE 6

Diagnostic test for identification of bacteria having acceptor structures showing specificity towards the structural element according to the invention.

a. Bacteria are incubated with sheep erythrocytes and human erythrocytes causing agglutination thereof. In a parallel experiment bacteria are incubated with sheep erythrocytes together with the structural element according to the invention at such concentration that

haemagglutination is completely inhibited. Haemagglutination and inhibition of haemagglutination is performed in accordance with Example 1. Positive haemagglutination of sheep erythrocytes and complete inhibition of haemagglutination after addition of oligosaccharide verifies the fact that the bacteria possess acceptor structure.

b. Bacteria are incubated with a composition wherein the structural element in question is covalently or through other association in multivalent form linked to a particular matrix according to Example 3 or 4. Incubation is performed on microscope slides for 10-15 minutes the preparation being then studied in microscope. If the bacteria possess acceptor structure the particles are covered with bacteria. With negative reaction the particles are free from bacteria.

c. Bacteria are admixed with a composition according to Example 3 or 4 on microscope slides, positive reaction resulting in agglutination of particles clad with the said structural element.

EXAMPLE 7

Purification of bacteria or acceptor structures

a. A composition according to Example 4 or 5 above is arranged in the form of a column. A mixture of bacteria is then passed through the column, bacteria possessing acceptor structures being held in the column by interaction with the receptor structures of the column. After rinsing the column can be eluted with buffer containing a receptor-active structural element according to the invention and this has for an effect that the bacteria with acceptor structures are eluted in a pure form.

b. By a process fully analogous to a. the acceptor structure can be obtained in a pure form.

Bacteria or acceptor structures can be used for

the manufacture of vaccins or for antibody analyses in for example body fluids, such as blood, urine or mother's milk.

EXAMPLE 8

Composition for use for the purpose of disinfection

By disinfection is here primarily intended removal of bacteria from a surface, for example a wound.

A 0.1 percent by weight aqueous solution of the compound β-D-Galp-(1-4)-D-GNAc is prepared and applied by means of a cotton pad onto a surface infected by N.gonorrhoeae. The solution results in effective removal of the bacterium from the surface.

EXAMPLE 9

The procedure of EXAMPLE 2 was repeated but in this case cells were collected from young healthy women by scraping the Buccal Mucosae with a wooden spatula. The bacterium used was N.meningitidis, international test strain 3K, Statens Seruminstitut, Copenhagen, Denmark.

In the experiments was found that 0.5 mg/ml of the oligosaccharide numbered (1) in EXAMPLE 2 reduced the number of adhering bacteria by about 15 %, whereas 5 mg/ml of (1) provided for a reduction of 90 %.

Encl. 1

Scheme 1 (synthesis of the α-OMe disaccharide 3)

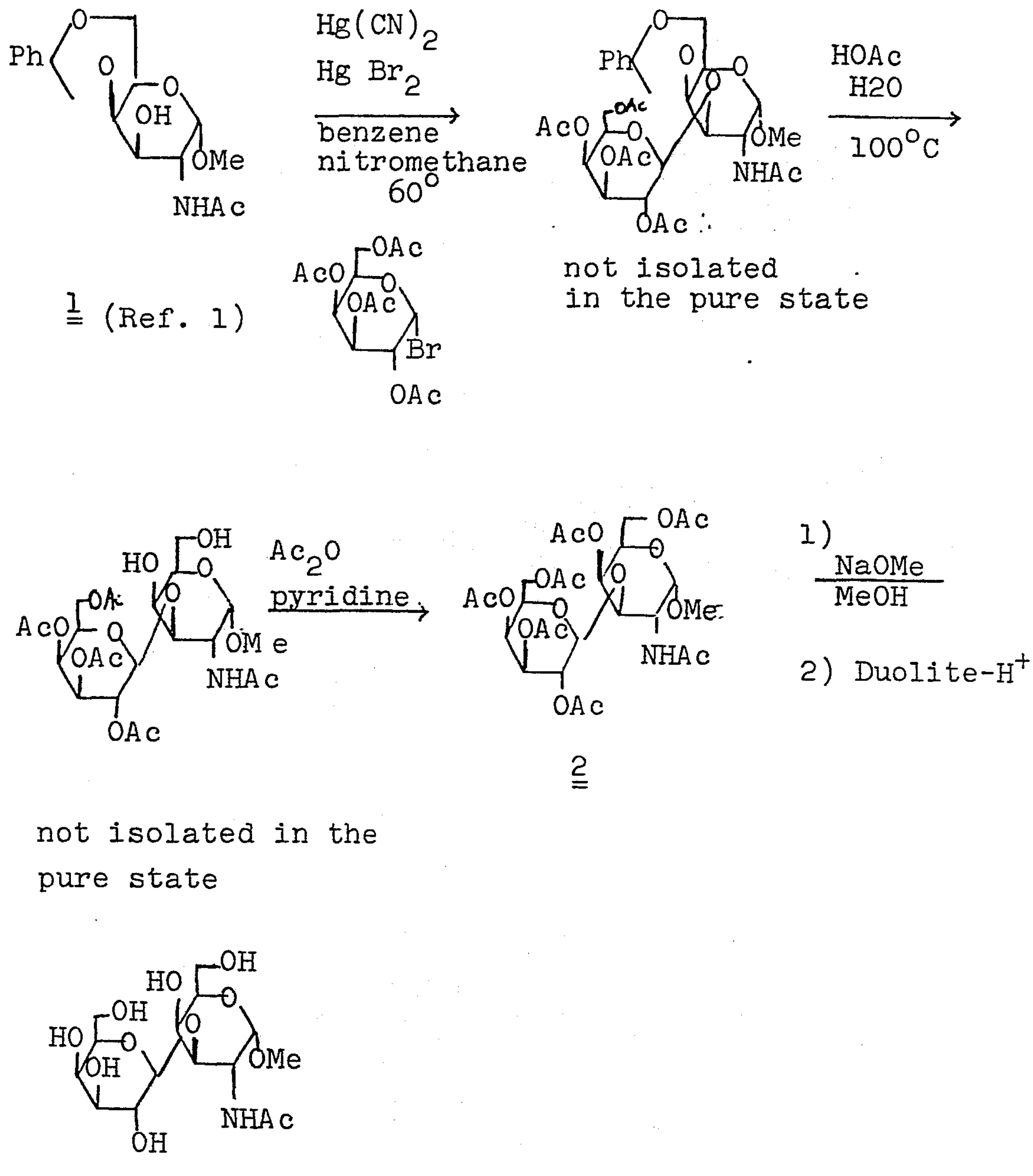


1 (Ref. 1)

not isolated in the pure state

2

not isolated in the pure state

3

Scheme 2 (synthesis of the β-OMe disaccharide 6)

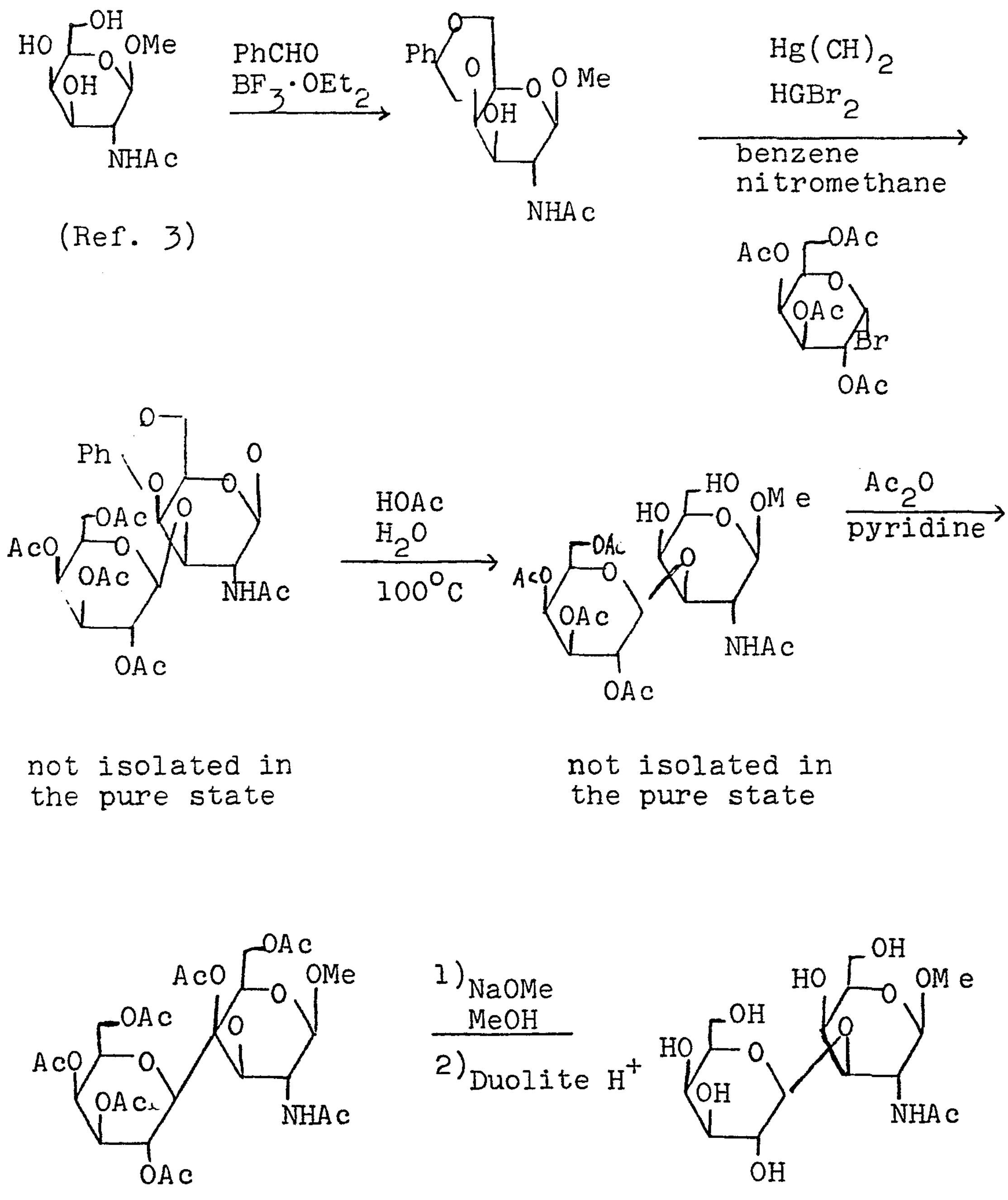


5

6

Methyl 2-acetamido-2-deoxy-3-O-(β-D-galactopyranosyl)-α--D-galactopyranoside (3)

From a mixture of methyl 2-acetamido-2-deoxy-4,6--O-benzylidene-α-D-galactopyranoside (Ref. 1) (646 mg, 2.00 mmoles), benzene (35 ml) and nitromethane (35 ml) approximately 25 ml of the solvent was distilled off in order to azeotropically remove moisture. After cooling acetobromogalactose (820 mg, 2.00 mmoles) together with mercuric cyanide (505 mg, 2.00 mmoles) and mercuric bromide (72 mg, 0.20 mmole) were added and the reaction mixture stirred at 60°C over night ( 15 h). Then another portion of acetobromo galactose (820 mg, 2.00 mmoles) and mercuric cyanide (505 mg, 2.00 mmoles) were added. (Ref. 2). The analysis ($SiO_2$, ethyl acetal:methanol 85:15) 3 h after this addition showed that all of the amino sugar aglycone was consumed and one major, faster running spot was present. The reaction mixture was diluted with methylene chloride (100 ml) and the organic solution washed with water (3x25 ml). After drying ($Na_2SO_4$) and removal of the solvent there remained 2.08 g of crude product, which was dissolved in 100 ml of 60 % acetic acid and the solution treated at 100°C for 45 min. The solvent was removed in vacuo and the residue was dried over phosphorus pentoxide and sodium hydroxide under vacuum. The dried product was acetylated with pyridine (20 ml) and acetic anhydride (10 ml) for 6 h at room temperature. The reagents were removed by azeotropic distillation with toluene (repeatedly) under vacuum. The residue was taken up in methylene chloride washed with 0.5 N HCl, saturated aqueous sodium bicarbonate and dried. After removal of the solvent in vacuo the crude per-acetylated disaccharide was chromatographed ($SiO_2$, ethylacetate : 1-butanol 95:5) and recrystallized from ethanol to give 505 mg (39 %) of the peracetylated title compound, 2, which

was pure by the ($SiO_2$ solvent as above) and NMR ($CDCl_3$) standards - Mp. 97°C . $(\alpha)_D^{23}$+ 69° (C 0.54, $CHCl_3$). 'H NMR($COCl_3$) δ 1.96, 1.99, 2.05, 2.06, 2.07, 2.12, 2.16 ($COCH_3$, 21H), 3.41 (S, 3H, $OCH_3$), 3.84 - 4.24 (m, 7H, H-5, H-5', H-6, H-6', H-3), 4.54 (m, 1H, H-2), 4.61 (d, 1H, H-1', $J_{1',2'}$ 7.9 Hz), 4.83 (d, 1H, H-1, $J_{1,2}$ 3.5 Hz), 5.16 (dd, 1H, H-2'), 5.39 (bd, 2H, H-4, H-4'), 5.64 (d, 1H, N-H). $^{13}$C NMR ($CDCl_3$) $\delta_{C-1}$ 98.7. ($J_{C-Haq}$174Hz), $\delta_{C-1'}$ 100.7 ($J_{C-Hax}$ 161Hz). The peracetylated title compound (170 mg, 0.262 mmole) was deacetylated with a catalytic amount of sodium methoxide in methanol (25-50 ml). After 4 h at room temperature the reaction mixture was neutralized with methanol washed and dried Duolite-$H^+$ ion exchange resin. After filtration and evaporation of the solvent these remained 100 mg (96 %) of the title compound (3), pure by the ($SiO_2$, chloroform:methanol:water 65:35:10) and NMR standards. 'HNMR ($D_2O$) δ 2.00 (s, 3H, N-$COCH_3$), 3.39 (s, 3H, $OCH_3$), 4.46 (d, 1H, H-1', $J_{1', 2'}$ 7.5Hz), 4.78 (d, 1H, H-1, $J_{1,2}$ 3.6Hz). 13CNMR ($D_2O$) δ 24.8 (CO-<u>$CH_3$</u>), 51.4 (C-2), 57.9 ($OCH_3$) 63.8, 64.0 (C-6, C-6'), 71.4, 71.6, 73.3, 73.4, 75.4, 77.8, 80.1 (C-H of the rings), 101.1 (C-1), 107.5 (C-1').

<u>Methyl 2-acetamido-2-deoxy-4,6-O-benzylidene-β-D--galactopyranoside</u> (4)

To a mixture of methyl 2-acetamido-2-deoxy-β-D--galactopyranoside (Ref. 3) (1.2 g, 5.1 mmoles) and benzaldehyde (1.20 ml 11.8 mmoles) borontrifluoride etherate (1.20 ml, 9.40 mmoles) was added at 0°C. The reaction mixture darkened and was stirred for 60 min. after which it was poured onto aq. sodium bicarbonate and 30 ml of ethyl acetate was added. Crystals were formed, which were collected and washed with a small amount of ethyl acetate. After drying the yield of the title compound was 0.60 g (38 %) of pure material (the $SiO_2$,

ethylacetate:methanol 85:15) 'HNMR ($CD_3OD$) δ 1.98 (s, 3H, NAc), 3.49 (s, 3H $OCH_3$), 3.54 (m, IH, H-5), 3.74 (dd, IH, H-3, $J_{3,4}$= 3.49 Hz, $J_{2,3}$= 108 Hz), 4.01 (dd, IH, H-z), 4.15 (dd, $H_Δ$-6) 4.24 (dd, $H_Δ$-6), 4.20 (bs, H-4), 4.39 (d, IH, H-1, $J_{1,2}$ = 8.36 Hz), 5.62 (s, IH, benzylidene), 7.34 (m, 3H aromatic), 7.56 (m, 2H, aromatic). $[α]_D^{23}$ + 9.6° (c 1.1, $CH_3OH$) mp 258°C.

Methyl 2-acetamido-2-deoxy-3-O-(β-D-galactopyranosyl)-β-D-galactopyranoside (6)

Following the same procedure as for the synthesis of the α-methyl glycoside isomer the title compound (297 mg, 37 %) was obtained from methyl 2-acetamido-2-deoxy-4,6-O-benzylidene-β-D-galactopyranoside (646 mg, 2.00 mmoles). 'HNMR ($D_2O$,+20°C) δ 2.00 (s, 3H,NHAc), 3.51 (s, $OCH_3$), 3.51 (H-2'[a]), 4.00 (dq, IH, H-2, $J_{23}$=10.6 Hz, $J_{1,2}$=8.2 Hz), 4.17 (bd, IH, H-4 or H-4'), 4.43 (bd, 2H, H-1 and H-1' $J_{1',2'}$= 7.1 Hz[b])

Methyl 2-acetamido-4,6-di-O-acetyl-2-deoxy-3-O-(2',3',4',6'-tetra-O-acetyl-β-D-galactopyranosyl)-β-D-galactopyranoside (5) was isolated (500 mg, 39 %) and used as a precursor for 6 in the preceding experiment. 'HNMR ($CDCl_3$) δ 1.96, 1.97, 2.05, 2.10, 2.13 (21 H, OAc and NHAc), 3.33 (m, IH, H-2), 3.50 (s, 3H, $OCH_3$), 4.62 (d, H-1', $J_{1',2'}$= 7.6 Hz), 4.68 (dd, H-3) 4.94 (d, H-1, $J_{1,2}$= 8.2 Hz), 4.97 (dd, H-3', $J_{3',4'}$= 3.1 Hz, $J_{2',3'}$= 10.2 Hz), 7.48 (dd, IH, H-2'), 5.34 (d, IH, H-4'), 5.42 (d, IH, H-4), 5.97 (d, IH, N-H, $J_{N-H,2}$ = 6.8 Hz).

a) Found by double resonance, b) at +50° the resonances of H-1 and H-1' separated.

When used in this disclosure the term "microorganism" is intended to cover bacteria, viruses, animal cells and plant cells.

References

1. P.J. Stoffyn and R.W. Jeanloz, J.Am.Chem.Soc. 1954, 76, 561.
2. The procedure is a standard one for the so called Helferich glycoside synthesis Cf. R.M.Ratcliffe, D.A. Baker and R.W. Lemieux, Carbohydr.Research, 1981, 93, 35.
3. T.L. Nagabhushan, Can.J.Chem., 1980, 58, 2720.
4. Svenson, S.B. and A.A. Lindberg (1979) J. Immunol. Meth. 25, 323.
5. Zopf, D. et al (1978 Immunol.Meth.enzymol. L, part C, 163.
6. Lönngren, J. et al (1976) Arch.Biochem. Biophys. 175, 661.
7. Gray, G.R. (1978). In Meth.enzymol. L, part C, 155.
8. McBroom, C.R., Samanen, C.H., Goldstein, I.J. In: Methods in Enzymology, Vol. 28B, ed. V. Ginsburg, p. 212. Academic Press, New York (1972).

0089939

CLAIMS

1. A composition for therapeutic or diagnostic use in connection with pathogenic microorganisms, characterized by containing as an active constituent a structural element having the formula:

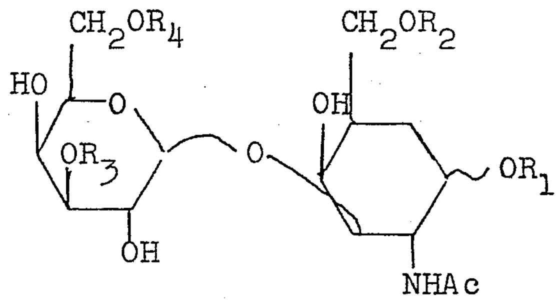

V

wherein $R_1$, $R_2$, $R_3$ and $R_4$ are same or different and are hydrogen or an organic residue, for example lower alkyl, lower acyl or a carbohydrate residue, or an inorganic residue, such as sulphate or phosphate, with the proviso that when $R_1$ is other than hydrogen $OR_1$ is in alpha-configuration, and that when $R_2=R_3=R_4=H$ $R_1$ is different from $-(CH_2)_nCOR$, where n=3-19 and R is an alkoxy, aryloxy, $NHNH_2$, OH or $N_3$ group, optionally in association with a pharmaceutically acceptable carrier.

2. A composition according to claim 1, characterized thereby that $R_2$, $R_3$ and $R_4$ are all hydrogen.

3. A composition according to claim 1 or 2, characterized thereby that $R_1$ is an amino acid or a polypeptide residue.

4. A composition according to claim 1 or 2, characterized thereby that $R_1$ is lower alkyl, for example methyl.

5. A process for therapeutic treatment of mammals including man, characterized by administering

to the mammal an active amount of a composition according to any preceding claim.

6. A composition according to any of claims 1-4 for use as an inhibitor of bacterial adherence to human cells.

7. A composition according to claim 1, wherein the bacterial adherence is that of a bacterium selected from the Genus Neisseria.

8. A composition according to claim 6, characterized by containing said structural element covalently linked to a macromolecular carrier, optionally via a coupling arm.

9. A composition according to claim 6 or 7, characterized by using as a macromolecular carrier a synthetic or naturally occurring polypeptide, polysaccharide, other polymer or particle.

10. A composition according to any of claims 6-8, characterized thereby that the coupling arm between structural element and macromolecular carrier is:

structural element - 1 - O - 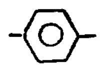 -NHCSNH-macromolecular carrier,

structural element -1-O- 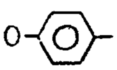 -N=N - macromolecular carrier,

structural element $-NH-(CH_2)_n-CH_2-$ 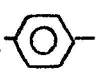 -NHCSNH-macromolecular carrier,

structural element $-NH-(CH_2)_n-CH_2-$ 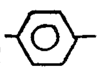 -N=N-macromolecular carrier,

structural element $-NH-(CH_2)_n-CO-NH-$macromolecular carrier,

structural element -NH-macromolecular carrier,
or
structural element $-NH(CH_2)_n-CO-NH-$macromolecular carrier,
wherein n can vary between 1 and 15.

11. A process for identification or quantification of the said structural element in native biological material from mammals including man, characterized by using in the process antibodies, the generation of which has been induced by the composition according to any of claims 1-4 and 6-10.

12. A process for the purification of acceptor structures of bacteria, characterized by using for the purification the affinity between the structural element of the composition according to any of claims 1-4 and 6-10 and the said acceptor structure on the bacteria.

13. A process for performing disinfection on surfaces, characterized by applying to the surface a composition according to any of claims 1-4 and 6-10 and then removing the composition with the bacteria adhering thereto.

European Patent Office

**PARTIAL EUROPEAN SEARCH REPORT**
which under Rule 45 of the European Patent Convention shall be considered, for the purposes of subsequent proceedings, as the European search report

0089939
Application number

EP 83 85 0077

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol.96, no.10, 1982, page 569, abstract no.160541n COLUMBUS OHIO (US) & Protides Biol. Fluids 1981 (Pub.1982).29th, 217-20 G.F. SPRINGER et al.: "Further evidence that terminal β-galactopyranosyl groups are immunodeterminant in blood group N specificity." * abstract * | 1,2,6-13 | A 61 K 31/70<br>C 07 H 3/04<br>A 61 K 39/00<br>G 01 N 33/48 |
| X | DE - A - 2 952 373 (E-Y LABORATORIES INC) * pages 1-8; page 17 * | | |
| X | FR - A - 2 487 984 (OTSUKA PHARMACEUTICALS CO) * pages 20-27 * ./. | 1,2,6 | TECHNICAL FIELDS SEARCHED (Int. Cl. 3)<br>A 61 K 31/00<br>C 07 H 3/00<br>G 01 N 33/00 |

**INCOMPLETE SEARCH**

The Search Division considers that the present European patent application does not comply with the provisions of the European Patent Convention to such an extent that it is not possible to carry out a meaningful search into the state of the art on the basis of some of the claims.
Claims searched completely: 1-4,6-13
Claims searched incompletely:
Claims not searched: 5
Reason for the limitation of the search:

Method for treatment of the human or animal body by surgery or therapy (see article 52(4) of the European Patent Convention).

| Place of search | Date of completion of the search | Examiner |
|---|---|---|
| The Hague | 04-05-1983 | VERHULST |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT



| DOCUMENTS CONSIDERED TO BE RELEVANT | | | CLASSIFICATION OF THE APPLICATION (Int. Cl.) |
|---|---|---|---|
| Category | Citation of document with indication, where appropriate, of relevant passages | Relevant to claim | |
| | -- | 13 | |
| X | CARBOHYDRATE RESEARCH, vol.86, 1980 Elsevier Scientific Publishing Co. AMSTERDAM (NL) H.J. ALLEN et al.; "Binding-Site Specificity of Lectins From Bauhinia purpurea alba, Sophora japonica And Wistaria Floribunda", pages 123-131 | | |
| | * page 126 * | 1,4, 6-13 | TECHNICAL FIELDS SEARCHED (Int. Cl.) |
| | -- | | |
| Y | EP - A - 0 060 999 (BEHRINGWERKE A.G.) | | |
| | * pages 24-27 * | 1,6-13 | |
| | -- | | |
| Y | EP - A - 0 044 188 (CHEMBIOMED) | | |
| | * pages 25-32 * | 1,6-13 | |
| | -- | | |
| Y | WO - A - 81 03175 (LEFFLER H.) | | |
| | * pages 1-6 * | 1,6-13 | |
| | -- | | |
| Y | EP - A - 0 035 484 (KÄLLENIUS G.) | | |
| | * pages 1-4 * | 1,6-13 | |
| | -- | | |
| A | CHEMICAL SOCIETY REVIEWS 1978, vol. 7, no.4, 1978 R.U. LEMIEUX, F.R.S.: "Haworth Memorial Lecture. Human Blood Groups and Carbohydrate Chemistry", pages 423-52 | | |
| | * pages 423-4 52 * | 1 | |
| | ----------- | | |